(19) Europäisches Patentamt
European Patent Office
Office européen des brevets

(11) Publication number : **0 503 827 A1**

(12) **EUROPEAN PATENT APPLICATION**

(21) Application number : **92301838.6**

(22) Date of filing : **04.03.92**

(51) Int. Cl.$^5$ : **A61K 37/24,** G01N 33/60, G01N 33/68, C07G 17/00, A61K 35/00

(30) Priority : **15.03.91 US 670536**

(43) Date of publication of application : **16.09.92 Bulletin 92/38**

(84) Designated Contracting States : **AT BE CH DE DK FR GB IT LI LU NL SE**

(71) Applicant : **PFIZER INC.**
**235 East 42nd Street**
**New York, N.Y. 10017 (US)**

(72) Inventor : **Kreutter, David K.**
**677 Summer Hill Road**
**Madison, Connecticut 06340 (US)**

(74) Representative : **Bradbrook, Geoffrey William et al**
**PFIZER LIMITED Ramsgate Road**
**Sandwich Kent, CT13 9NJ (GB)**

(54) **Amylin and calcitonin gene-related peptide use and antagonists thereof.**

(57) This invention relates to the use of amylin or calcitonin gene-related peptide (CGRP) to reduce insulin stimulated glycolysis in muscle or muscle cells. This invention also relates to a method for assaying the ability of a compound to inhibit the reduction in insulin stimulated glycolysis in muscle or muscle cells caused by amylin or CGRP. Further, this invention relates to compounds capable of inhibiting the reduction in insulin stimulated glycolysis in muscle or muscle cells caused by amylin or CGRP. This invention also relates to pharmaceutical compositions and methods for inhibiting the reduction in insulin stimulated glycolysis in muscle or muscle cells in a mammal caused by amylin or CGRP.

EP 0 503 827 A1

This invention relates to the use of amylin or calcitonin gene-related peptide (CGRP) to reduce insulin stimulated glycolysis in muscle or muscle cells. Further, this invention relates to a method for assaying the ability of a compound to inhibit the reduction in insulin stimulated glycolysis in muscle or muscle cells caused by amylin or CGRP. Particular methods within the scope of this invention include methods for assaying the ability of a compound to inhibit the reduction in insulin stimulated glycolysis in muscle or muscle cells caused by human or rat amylin. Further still, this invention relates to compounds capable of inhibiting the reduction in insulin stimulated glycolysis in muscle or muscle cells caused by amylin or CGRP. Yet further still, this invention relates to pharmaceutical compositions and methods for inhibiting the reduction in insulin stimulated glycolysis in muscle or muscle cells in a mammal caused by amylin or CGRP.

Amylin is a 37 amino acid peptide of molecular weight 3905 daltons that is a secretory product of pancreatic β cells. Westermark, P., et al., Biochem. Biophys. Res. Commun. 140:827-831 (1986); Clark, A., et al., Lancet ii:231-234 (1987); Westermark, P., et al., Diabetologia 30:887-892 (1987); Cooper, C. J. S., et al., Proc. Natl. Acad. Sci. USA 84:8628-8632 (1987); Westermark, P., et al., Proc. Natl. Acad. Sci. USA 84:3881-3885 (1987). In this type II diabetic, there are extensive deposits of amylin derived amyloid in the pancreas and it has been postulated that the amyloid contributes to the pancreatic pathology associated with the disease. Westermark, P., et al., Biochem. Biophys. Res. Commun. 140:827-831 (1986); Clark, A., et al., Lancet ii:231-234 (1987); Westermark, P., et al., Diabetologia 30:887-892 (1987); Cooper, C. J. S., et al., Proc. Natl. Acad. Sci. USA 84:8268-8632 (1987); Westermark, P., et al., Proc. Natl. Acad. Sci. USA 84:3881-3885 (1987); Westermark, P., et al., Am. J. Pathol. 127:414-417 (1987); Clark, A., et al., Diabetologia 33:285-289 (1990).

In isolated soleus muscle, amylin suppresses insulin-stimulated glycogen synthesis. Leighton, B., et al., Nature 335:632-635 (1988); Cooper, G. J. S., et al., Proc. Natl. Acad. Sci. USA 85:7763-7766 (1988). The effect of amylin on insulin action in skeletal muscle in vivo has been reported to be the inhibition of glycogenesis which is the major effect in isolated soleus muscle described above.

The amino acid sequence for human amylin is disclosed in EP 289 287, published November 2, 1988. The amino acid sequence for rat amylin is disclosed in Asai, J., et al., Biochem. Biophys. Res. Commun. 164:400-405 (1989). The teachings thereof are incorporated herein by reference.

Certain amylin antagonists and the use thereof are described in WO89/0613 (PCT/US89/00049), published July 13, 1989.

Calcitonin gene-related peptide (CGRP) is known to occur in at least two forms (α and β) in humans, each of which is comprised of 37 amino acids. The β form of CGRP is 46% homologous with amylin. Westermark, P., et al., Biochem. Biophys, Res. Commun. 140:827-831 (1986) and Westermark, P., et al. Proc. Natl. Acad. Sci. USA 84:3881-3885 (1987).

Until the invention herein, there has been no report of the ability of amylin or CGRP to reduce insulin stimulated glycolysis in muscle cells.

This invention relates to the use of amylin or CGRP to reduce insulin stimulated glycolysis in muscle or muscle cells. As used throughout this Specification and the appendant claims, the term amylin includes human amylin, rat amylin and C-terminal amidated forms thereof. Also included within the term amylin are polypeptides having substantially the same amino acid sequence and substantially the same activity as any of said forms of amylin. The term calcitonin gene-related peptide (CGRP) includes the known α and β polypeptides as well as polypeptides having substantially the same amino acid sequence and substantially the same activity as that of the known polypeptides. Further, the term muscle includes intact muscle in vivo as well as various ex vivo preparations of muscle such as strips thereof. The term muscle cells is included for completeness and includes all muscle cells whether present in intact muscle in vivo, various ex vivo preparations of muscle or in in vitro cultures of muscle cells per se. The use of amylin or CGRP to reduce insulin stimulated glycolysis in muscle or muscle cells is of particularly beneficial utility in the search for compounds which inhibit the ability of amylin or CGRP to reduce insulin stimulated glycolysis and, thereby, are of benefit in the treatment of Type II (NIDDM) diabetes.

This invention also relates to a method for assaying the ability of a compound to inhibit the reduction in insulin stimulated glycolysis in muscle or muscle cells caused by amylin or CGRP. The method comprises incubating muscle or muscle cells in the presence of insulin, amylin or CGRP, a compound and radiolabeled glucose; measuring the amount of radiolabeled glycolysis product of the so incubated muscle or muscle cells; and comparing the amount so measured to the amount of radiolabeled glycolysis measured product when muscle or muscle cells are incubated in the presence of insulin, amylin or CGRP and radiolabeled glucose.

For the purposes of the above described method it is preferable to employ rat soleus muscle which has been dissected and stripped into pieces weighing about 25-35 mg according to the procedure described by Crettaz, M., et al., Biochem. J. 186:525-534 (1980). The muscle pieces are placed in appropriate vessels containing gassed (95% $O_2$/5% $CO_2$) Krebs-Ringer bicarbonate buffer containing 1.2 mM calcium. An appropriate vessel for such muscle pieces is a 50 ml Erlenmeyer flask containing 4 ml of the gassed Krebs-Ringer bicarbonate buffer containing 1.2 mM calcium as described above. While not essential to the practice of the method of this invention, it is preferable to incubate the muscle pieces in said buffer at 37°C (shaking water bath) for 15 minutes under an atmosphere of 95% $O_2$/5% $CO_2$ followed by incubation for 15 minutes at 37°C (shaking water bath) in said buffer which additionally contains insulin, amylin and a compound to be assayed prior to incubation at 37°C, in the presence of insulin, amylin, radiolabeled glucose and a compound to be assayed. Each of such subsequent incubations are preferably carried out in 4 ml of the respective buffer in 50 ml Erlenmeyer flasks.

Incubation of the muscle pieces in said buffer containing insulin, amylin or CGRP, radiolabeled glucose and a compound to be assayed is conducted at 37°C (shaking water bath) where the buffer is gassed (95% $O_2$/5% $CO_2$) for the first five minutes followed by 55 minutes, also at 37°C (shaking water bath) with stoppered vessels. For the purpose of the above method, it is preferable to use insulin at a level of about $10^{-10}$ M to $10^{-7}$ M. It also is preferable to use human or rat amylin and the level thereof which is preferable is $10^{-10}$ M to $10^{-7}$ M. When CGRP is used, it is preferable to use a level of from about $10^{-10}$ to $10^{-7}$. While a variety of labels can be used for the radiolabeled glucose employed in this invention, it is a requirement that such glucose be radiolabeled in such a manner that, upon glycolysis, a detectable radiolabeled glycolysis product is produced. Thus, it is preferable to use D-[3-$H^3$]glucose in the method of this invention. Further, it is still more preferable to use D-[3-$H^3$]glucose at 0.5 μCi/ml of the buffer.

The amylin and CGRP used in this invention can be made according to standard protein synthesis techniques well known to those skilled in the art. Further, amylin and CGRP can be produced using recombinant DNA technology. Yet further still, amylin and CGRP can be obtained commercially from Peninsula Laboratories, Inc., 611 Taylor Way, Belmont, California 94002 or Sigma Chemical Co., P. O. Box 14508, St. Louis, Missouri 63178. Insulin is commercially available and can be obtained from various commercial sources such as Elanco Products Co., Indianapolis, Indiana 46285. Radiolabeled glucose is also commercially available from various suppliers such as Amersham Corp., 2636 South Clearbrook, Arlington Heights, Illinois 60065 and New England Nuclear, P. O. Box 80024, Wilmington, Delaware 19880.

Following completion of the incubation period in the presence of radiolabeled glucose, the muscle pieces are removed and the incubation buffer is assayed for radiolabeled glycolysis product. Since it is preferable to employ D-(3-$H^3$]glucose in the method described above, it is preferable to assay the incubation buffer for $^3H_2O$ as described by Ashcroft, et al., Biochem. J. 126:525-532 (1972). To assay for $^3H_2O$, it is preferable to place 100 μl of the incubation buffer and 10 μl 1N HCl into a 1.5 microcentrifuge tube without a cap, then place that tube into a scintillation vial containing 1 ml $H_2O$. The vial is then capped tightly and incubated for about 18-24 hours at 60°C to allow equilibration of $^3H_2O$ between the tube and the vial. Then, the tube is removed from the vial and the vial is counted for $^3H$. Appropriate controls for assay of $^3H_2O$ glycolysis product are tubes containing 100 μl $^3H_2O$ (50,000-100,000 cpm) and 10 μl 1N HCl for correcting for the completeness of the $^3H_2O$ equilibration between the tube and the vial; and buffer containing insulin, amylin, radiolabeled glucose and the compound but not exposed to muscle for use in determining background from the assay.

It is preferable to assay duplicate samples from each muscle incubation buffer and to assay three to four $^3H_2O$ equilibration and background controls. To calculate the amount of glycolysis in the incubation buffer, employing the preferable volumes and times above, the following equation is used:

$$\frac{(Y)\ (44)}{\left(\begin{array}{l}\text{Specific activity}\\ \text{of D-[3-H}^3\text{]glucose}\\ \text{expressed as cpm/nmol}\end{array}\right)(Z)\ \left(\begin{array}{l}\text{mg wet weight}\\ \text{soleus muscle}\end{array}\right)} = \begin{array}{l}\text{glycolysis}\\ \text{(nmol/hr/}\\ \text{mg wet wt.)}\end{array}$$

where Y equals sample $^3H$ cpm minus background cpm and Z equals percent completion of $^3H_2O$ equilibrium.

Of course, the method described above can be applied mutatis mutandis using different volumes and vessels as well as muscle cells instead of muscle pieces. One skilled in the art, enabled by the disclosure herein, will readily appreciate such variations and make the appropriate changes to the equation above such that the measure of glycolysis can be calculated for such variations. Further, this invention is not limited to the determination of glycolysis as expressed in nmol/hr/mg wet weight. All other appropriate measurements of glycolysis are within the scope of this invention.

A compound which inhibits the ability of amylin or CGRP to reduce insulin stimulated glycolysis in muscle or muscle cells is identified by comparing the level of glycolysis, for example $^3H$ cpm, present in the incubation buffer when the compound is present with the level when the compound is absent. For those compounds which result in a higher level glycolysis product, for example a larger number of $^3H$ cpm, then such compounds inhibit

the ability of amylin or CGRP to reduce insulin stimulated glycolysis in muscle or muscle cells.

Using the above described method but not employing a compound to inhibit the effect of amylin, the data shown in Table I, below, were obtained.

Table I

| Soleus muscle (mg) | Rat amylin (M) | Human amylin (M) | Insulin ($10^{-8}$M) | Glycolysis (nmol/hr/ mg wet wt.) |
|---|---|---|---|---|
| 32.0 | - | - | - | 10.1 |
| 23.9 | - | - | + | 17.0 |
| 33.8 | - | - | - | 8.18 |
| 28.1 | - | - | + | 13.0 |
| 24.2 | $10^{-10}$ | - | + | 16.0 |
| 34.4 | $10^{-10}$ | - | + | 10.6 |
| 31.7 | $10^{-9}$ | - | + | 12.5 |
| 34.4 | $10^{-9}$ | - | + | 11.4 |
| 26.3 | $10^{-8}$ | - | + | 13.8 |
| 24.2 | $10^{-8}$ | - | + | 13.1 |
| 30.5 | $10^{-7}$ | - | + | 12.0 |
| 34.1 | $10^{-7}$ | - | + | 11.8 |
| 28.2 | - | $10^{-10}$ | + | 8.47 |
| 34.2 | - | $10^{-10}$ | + | 8.28 |
| 29.8 | - | $10^{-9}$ | + | 9.37 |
| 35.3 | - | $10^{-9}$ | + | 8.38 |
| 24.9 | - | $10^{-8}$ | + | 8.49 |
| 25.2 | - | $10^{-8}$ | + | 11.8 |
| 26.7 | - | $10^{-7}$ | + | 11.4 |
| 30.8 | - | $10^{-7}$ | + | 7.5 |

Again using the above described method but not employing a compound to inhibit the effect of the C-terminal amidated amylin (synthesized by Glenn C. Andrews of Pfizer Inc.) and human amylin (Penisula Labs. Cat. #7321), the data shown in Table II, below were obtained.

Table II

| Soleus muscle (mg) | Insulin ($10^{-8}$M) | C-term. amidated amylin (M) | Human amylin (M) | Glycolysis (nmol/hr/ mg wet wt.) |
|---|---|---|---|---|
| 34.8 | - | - | - | 9.5 |
| 26.8 | + | - | - | 12.1 |
| 30.9 | - | - | - | 6.72 |
| 30.3 | + | - | - | 11.5 |
| 31.9 | + | $10^{-11}$ | - | 7.86 |
| 33.8 | + | $10^{-11}$ | - | 10.1 |
| 32.2 | + | $10^{-10}$ | - | 10.8 |
| 26.5 | + | $10^{-10}$ | - | 12.5 |
| 28.2 | + | $10^{-9}$ | - | 8.86 |
| 30.7 | + | $10^{-9}$ | - | 9.80 |
| 30.2 | + | $10^{-8}$ | - | 8.10 |
| 31.1 | + | $10^{-8}$ | - | 7.27 |
| 36.0 | + | $10^{-7}$ | - | 5.43 |
| 33.2 | + | $10^{-7}$ | - | 5.71 |
| 33.1 | + | - | $10^{-9}$ | 7.23 |
| 31.6 | + | - | $10^{-9}$ | 5.91 |
| 33.0 | + | - | $10^{-8}$ | 5.94 |
| 23.2 | + | - | $10^{-8}$ | 6.54 |
| 27.4 | + | - | $10^{-7}$ | 7.46 |
| 35.4 | + | - | $10^{-7}$ | 6.49 |

Further still, when the above described method was used but not employing a compound to inhibit the effect of rat CGRP (Sigma Cat. #C 0167), the data shown in Table III, below, were obtained.

Table III

| Soleus muscle (mg) | Insulin ($10^{-8}$M) | CGRP (M) | Glycolysis (nmol/hr/ mg wet wt.) |
|---|---|---|---|
| 23.8 | - | - | 11.3 |
| 24.9 | + | - | 18.2 |
| 28.0 | - | - | 14.7 |
| 29.9 | + | - | 12.4 |
| 32.5 | + | $10^{-9}$ | 11.8 |
| 31.3 | + | $10^{-9}$ | 14.6 |
| 33.7 | + | $10^{-8}$ | 5.76 |
| 26.7 | + | $10^{-8}$ | 7.21 |
| 30.5 | + | $10^{-7}$ | 6.49 |
| 34.8 | + | $10^{-7}$ | 6.05 |

## Claims

1. A method for using amylin or calcitonin gene-related peptide to reduce insulin stimulated glycolysis in muscle or muscle cells which comprises incubating muscle or muscle cells in the presence of an insulin stimulated glycolysis reducing amount of amylin or calcitonin gene-related peptide.

2. The method according to claim 1 wherein the amylin is human or rat amylin.

3. A method for assaying the ability of a compound to inhibit the reduction in insulin stimulated glycolysis in muscle or muscle cells caused by amylin or calcitonin gene-related peptide, which method comprises:
   (a) incubating muscle or muscle cells in the presence of insulin, amylin or calcitonin gene-related peptide, the compound and radiolabeled glucose;
   (b) measuring the amount of radiolabeled glycolysis product of the incubated muscle or muscle cells of step (a); and
   (c) comparing the amount measured in step (b) to the amount measured by the method of step (b) when muscle or muscle cells are incubated in the presence of insulin, amylin or calcitonin gene-related peptide and radiolabeled glucose.

4. The method according to claim 3 wherein the amylin is human or rat amylin and the muscle is rat soleus muscle.

5. The method according to claim 4 wherein the insulin is present at about $10^{-10}$ M to about $10^{-7}$ M.

6. The method according to claim 5 wherein the human amylin is present at about $10^{-10}$ M to about $10^{-7}$ M and the rat amylin is present at about $10^{-10}$ M to about $10^{-7}$ M.

7. The method according to claim 6 wherein the radiolabeled glucose is tritiated glucose.

8. The method according to claim 3 wherein the muscle or muscle cells are preincubated in the presence of insulin, amylin or calcitonin gene-related peptide, the compound and glucose prior to step (a).

9. A compound, identified according to the method of claim 3, capable of inhibiting the reduction in insulin stimulated glycolysis in muscle or muscle cells caused by amylin or calcitonin gene-related peptide.

**10.** Use of a compound according to claim 9 for making a medicament for inhibiting the reduction in insulin - stimulated glycolysis in muscle or muscle cells caused by amylin or calcitonin gene-related peptide.

European Patent Office

# EUROPEAN SEARCH REPORT

Application Number

EP 92 30 1838

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | WO-A-8 906 135 (AMYLIN CORPORATION)<br>* the whole document *<br>--- | 1-10 | A61K37/24<br>G01N33/60<br>G01N33/68<br>C07G17/00<br>A61K35/00 |
| X | EP-A-0 408 294 (AMYLIN CORPORATION)<br>* the whole document *<br>--- | 1-10 | |
| X<br>A | EP-A-0 309 100 (AMYLIN CORPORATION)<br>* the whole document *<br>--- | 1,2<br>3-10 | |
| D,A | EP-A-0 289 287 (AMYLIN CORPORATION)<br>* the whole document *<br>--- | 1-10 | |
| P,X | FEBS LETTERS.<br>vol. 296, no. 2, 20 January 1992, AMSTERDAM NL<br>pages 123 - 127;<br>A. CHANTRY ET AL.: 'Biotinyl analogues of amylin as biologically active probes for amylin/CGRP receptor recognition.'<br>* abstract *<br>----- | 1-10 | |
| | | | TECHNICAL FIELDS SEARCHED (Int. Cl.5 ) |
| | | | A61K<br>C07K<br>G01N |

The present search report has been drawn up for all claims

| Place of search | Date of completion of the search | Examiner |
|---|---|---|
| THE HAGUE | 01 JULY 1992 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)